# FASCICULE DE BREVET EUROPEEN

(11) **EP 3 701 802 B1**
(45) Date de publication et mention de la délivrance du brevet: **04.12.2024**
(21) Numéro de dépôt: 20159432.2
(22) Date de dépôt: 25.02.2020
(51) Int. Cl.: A23J 1/10, A23J 3/34, A23K 20/142

(54) **COMPOSITION À HAUTES TENEURS EN ACIDES AMINÉS LIBRES ET UTILISATION EN TANT QUE MATIÈRE PREMIÈRE ET ALIMENT COMPLET POUR L'ALIMENTATION ANIMALE**
ZUSAMMENSETZUNG MIT EINEM HOHEN GEHALT AN FREIEN AMINOSÄUREN, UND VERWENDUNG ALS ROHSTOFF UND VOLLWERTIGES NAHRUNGSMITTEL FÜR DIE TIERERNÄHRUNG
COMPOSITION WITH HIGH FREE AMINO ACID CONTENT AND USE AS RAW MATERIAL AND COMPLETE FOOD FOR ANIMAL NUTRITION

(30) Priorité: 28.02.2019 FR 1902112
(43) Date de publication de la demande: 02.09.2020
(73) Titulaire: Bretagne Chimie Fine, 56140 Pleucadeuc (FR)
(72) Inventeur: DUPERRAY, Joel, 56250 TREFFLEAN (FR); SERGHERAERT, Renaud, 56870 BADEN (FR)
(74) Mandataire: Oak & Fox

(56) Documents cités:
- DD-A1- 244 760
- JP-A- H0 311 099
- US-A1- 2010 202 936
- US-A1- 2016 157 511
- US-A1- 2018 263 259
- STEIN J ET AL: "Amino acids -Guidelines on Parenteral Nutrition, Chapter 4", GMS GERMAN MEDICAL SCIENCE, 18 November 2009 (2009-11-18), XP093172239, Retrieved from the Internet <URL:https://www.ncbi.nlm.nih.gov/pmc/articles/PMC2795371/pdf/GMS-07-24.pdf>
- ERIKSSON S ET AL: "A COMPARISON OF THE EFFECTS OF INTRAVENEOUS INFUSION OF INDIVIDUAL BRANCHED-CHAIN AMINO ACIDS ON BLOOD AMINO ACID LEVELS IN MAN", CLINICAL SCIENCE, BIOCHEMICAL SOCIETY AND THE MEDICAL RESEARCH SOCIETY, LONDON, GB, vol. 60, no. 1, 1 January 1981 (1981-01-01), pages 95 - 100, XP008002556, ISSN: 0143-5221

## Description

La présente invention se rapporte au domaine des compositions à hautes teneurs en acides aminés libres obtenues à partir d'hydrolysats de kératine et à leur utilisation en tant que matières premières et aliment complet pour l'alimentation animale.

Les compositions à base d'acides aminés sont utilisées dans des domaines très divers tels que la nutraceutique, la cosmétique, la nutrition humaine et animale, pour des applications très différentes et spécifiques dans chacun de ces domaines. On peut notamment citer les utilisations visant la croissance et la brillance des cheveux, l'amélioration du sommeil et la réduction du stress, ainsi que des utilisations visant le rééquilibrage en acides aminés essentiels et comme source de protéines en nutrition infantile et également en nutrition canine et féline.

Une des façons d'obtenir une composition à base d'acides aminés est de réaliser un hydrolysat de matières kératiniques.

Les matières kératiniques naturelles comprennent majoritairement des polypeptides de haut poids moléculaire et à la structure très réticulée la rendant peu accessible aux enzymes. Cette matière kératinique naturelle est peu digestible. Cependant, il est connu que l'hydrolyse des matières kératiniques en acides aminés permet d'en améliorer la digestibilité.

Les hydrolysats de kératine proposés à la vente notamment à titre de compléments alimentaires, d'ingrédients de formulation de recettes en nutrition animale ou de matière première pour l'alimentation animale sont généralement obtenus par hydrolyse très partielle. Ces hydrolysats présentent généralement une masse moléculaire élevée due à la présence de taux élevés d'acides aminés dits « liés » formant des peptides. Typiquement la masse moléculaire des compositions du commerce est d'au moins 5 000 daltons. Ces hydrolysats de kératine sont relativement peu digestibles et contiennent très peu voire pas d'acides amines libres. En effet, il est techniquement difficile et coûteux sur le plan industriel d'obtenir un hydrolysat de kératine présentant un taux d'acides aminés libres très élevé. En outre une hydrolyse trop poussée présente un risque de dénaturation et de destruction des acides aminés.

L'obtention de tels hydrolysats requiert un savoir-faire basé sur l'utilisation et la maîtrise d'entrants chimiques concentrés ainsi que de procédés relevant du domaine du génie chimique. Ce haut niveau d'exigences techniques explique que la majorité des hydrolysats protéiques présents sur le marché sont issus d'une hydrolyse partielle et moins complète se traduisant par une proportion plus faible en acides aminés libres.

Or, comme présenté dans l'article « Extensive protein hydrolyzation is indispensable to prevent IgE-mediated poultry allergen recognition in dogs and cats » Olivry et al. BMC Veterinary Research (2017) 13 :251, en fonction de l'application souhaitée, il peut être avantageux voire nécessaire de disposer de compositions à très haute teneur en acides aminés libres.

La demande de brevet JP H0311099A décrit des hydrolysats de kératine obtenus en deux étapes : une étape d'hydrolyse acide et/ou enzymatique conduisant à l'obtention d'un hydrolysat de poids moléculaire compris entre 300 et 5000 Da et un taux de cystine compris entre 5 et 18% molaire par rapport à l'ensemble des acides aminés puis une étape de réduction par électrolyse conduisant à l'obtention d'un hydrolysat de poids moléculaire compris entre 200 et 3000 Da et un taux de cystéine compris entre 5 et 18% molaire par rapport à l'ensemble des acides aminés ainsi que leur utilisation en cosmétique capillaire. L'hydrolysat selon la présente invention ne comprend pas de cystéine, en outre il ne présente pas un taux de cystine si élevé.

De manière surprenante et avantageuse les auteurs de la présente invention sont parvenus à surmonter les problèmes de l'art antérieur et ont obtenu un hydrolysat de kératine dont la quasi-totalité des acides aminés sont des acides aminés libres, en particulier les acides aminés parmi les plus difficiles à obtenir sous forme libre lors du procédé d'hydrolyse tels que la valine, la leucine, l'isoleucine sont obtenus sous forme libre. En outre, les acides aminés libres obtenus selon l'invention ne sont pas endommagés ni dénaturés, en particulier la valine, la leucine, l'isoleucine.

D'autres aspects, avantages, propriétés de la présente invention sont présentés dans la description et les exemples qui suivent.

La présente invention, qui est définie par les revendications, a pour objet un hydrolysat de kératine de volaille comprenant au moins 94 %, de préférence au moins 96 %, et de manière encore préférée 100% en poids d'acides aminés libres par rapport au poids total des acides aminés de l'hydrolysat, ledit hydrolysat comprenant au moins les acides aminés suivants :
au moins 93%, de préférence au moins 95%, de valine sous forme libre en poids par rapport au poids total de valine dans l'hydrolysat, au moins 90%, de préférence au moins 95%, d'isoleucine sous forme libre en poids par rapport au poids total d'isoleucine dans l'hydrolysat, et au moins 95%, de préférence 100%, de leucine sous forme libre en poids par rapport au poids total de leucine dans l'hydrolysat Selon l'invention cet hydrolysat est susceptible d'être obtenu par le procédé décrit plus loin.

De préférence, l'hydrolysat selon la présente invention comprend moins de 2%, de préférence moins de 1 % et de manière encore préférée moins de 0,5 % en mole de cystine par rapport au nombre total de moles d'acides aminés de l'hydrolysat, de manière encore préférée, l'hydrolysat ne contient pas de cystine.

Selon l'invention, l'hydrolysat de kératine est un hydrolysat de matière kératinique de volaille.

Un deuxième objet de la présente invention vise un procédé de préparation de l'hydrolysat selon l'invention dans lequel la matière kératinique est une matière kératinique de volaille, comprenant au moins les étapes suivantes, dans cet ordre :
- soumettre la matière kératinique à au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton, ladite hydrolyse chimique étant effectuée en deux étapes:
- une première hydrolyse chimique réalisée à une température allant de 60 à 80 °C pendant une période allant de 4 à 5 heures puis
- une deuxième hydrolyse chimique réalisée à une température allant de 100 à 115°C pendant une période allant de 5 à 8 heures,
   les deux hydrolyses pouvant être réalisées sans étape de pause intermédiaire ou en effectuant une étape de pause intermédiaire comprise entre 1 heure et 7 jours;
- extraire la tyrosine et la cystine dudit hydrolysat de préférence au moyen d'une base;
   - dessaler ledit hydrolysat ; -effectuer une nanofiltration au moyen d'une membrane filtrante de calibre maximal 300 Da, à une pression comprise entre 15 et 40 bars et à un débit allant de 300 à 900 L/h
- éventuellement sécher. Les hydrolysats de kératine comprenant au moins 94% en poids d'acides aminés libres par rapport au poids total des acides aminés de l'hydrolysat selon l'invention sont susceptibles d'être obtenus par ce procédé.

La présente invention vise en outre une matière première pour l'alimentation animale comprenant de 80 à 100 % en poids de l'hydrolysat selon l'invention par rapport au poids total de ladite matière première.

La présente invention vise encore un aliment complet pour l'alimentation animale comprenant de 5 à 40 % en poids de l'hydrolysat selon l'invention par rapport au poids total dudit aliment complet.

La composition selon l'invention, qui est avantageusement un hydrolysat de kératine, présente une teneur très élevée en acides aminés libres qui lui confère des propriétés spécifiques notamment des propriétés nutritionnelles avantageuses, une biodisponibilité élevée voire très élevée, des propriétés anallergiques, ainsi qu'un pouvoir d'appétence pour les espèces animales terrestres et aquatiques.

Un avantage de la composition selon la présente invention est qu'elle est très digestible. La composition selon l'invention présente une digestibilité vraie des acides aminés très proche du maximum possible (100%), mesurée selon la méthode décrite par par Cozannet P., Primot Y., Gady C., Métayer J.P., Lessire M., Skiba F., Noblet J. dans « Standardised amino acid digestibility of wheat distillers' dried grains with solubles in force-fed cockerels ». British Poultry Science, Feb. 2011 ; 52(1) :72-81. Cette haute digestibilité permet d'utiliser la composition selon l'invention dans les domaines alimentaire et agroalimentaire. La composition selon l'invention est également soluble dans l'eau, en effet 1 g de composition selon l'invention est soluble dans 5 mL d'eau. Cette solubilité lui confère des propriétés intéressantes pour la mise en œuvre et le développement de caractéristiques organoleptiques au sein du produit final, en particulier un pouvoir d'appétence élevé. En particulier le taux très faible de cystine dans l'hydrolysat contribue à le rendre très hydrosoluble.

L'hydrolysat, selon la présente invention se distingue par son taux élevé d'acides aminés sous forme libre : au moins 94%, de préférence 96%, avantageusement 100% en poids des acides aminés présents dans l'hydrolysat selon l'invention sont sous forme libre.

L'hydrolysat selon l'invention comprend en outre :
au moins 93%, de préférence au moins 95%, de valine sous forme libre en poids par rapport au poids total de valine dans l'hydrolysat,
au moins 90%, de préférence au moins 95%, d'isoleucine sous forme libre en poids par rapport au poids total d'isoleucine dans l'hydrolysat,
au moins 95%, de préférence 100%, de leucine sous forme libre en poids par rapport au poids total de leucine dans l'hydrolysat.

. L'hydrolysat selon l'invention est obtenu à partir de matières kératiniques naturelles, qui sont de volaille, avantageusement à partir de plumes de volailles. A titre de volailles, on peut citer les poules, poulets, dindes, canards, oies...

En particulier, l'hydrolysat selon la présente invention n'est pas obtenu à partir de kératine humaine tels que les cheveux.

Avantageusement, la teneur en acides aminés de l'hydrolysat selon l'invention va de 40% à 90%, de préférence 45% à 87% en poids par rapport au poids total de l'hydrolysat, l'hydrolysat comprenant en outre de la matière minérale et de l'eau. Comme déjà mentionné les acides aminés de l'hydrolysat selon l'invention sont essentiellement des acides aminés libres.

L'hydrolysat selon la présente invention comprend au moins 94%, de préférence 96%, avantageusement 100% en poids d'acides aminés libres par rapport au poids total des acides aminés de l'hydrolysat. Les acides aminés libres ne sont pas dénaturés. En outre l'hydrolysat selon l'invention présente des taux élevés en acides aminés ramifiés libres : valine, leucine et isoleucine.

Or ces acides aminés ramifiés sont connus pour être plus difficiles à libérer dans des conditions de mise en oeuvre identiques.

Avantageusement, l'hydrolysat selon l'invention comprend les acides aminés libres suivants :
au moins 95%, de préférence 100%, de thréonine sous forme libre en poids par rapport au poids total de thréonine dans l'hydrolysat ;
au moins 95%, de préférence 100%, de sérine sous forme libre en poids par rapport au poids total de sérine dans l'hydrolysat ;
au moins 95%, de préférence 100%, de glycine sous forme libre en poids par rapport au poids total de glycine dans l'hydrolysat ;
au moins 95%, de préférence 100%, d'alanine sous forme libre en poids par rapport au poids total d'alanine dans l'hydrolysat ;
au moins 95%, de préférence 100%, de phénylalanine sous forme libre en poids par rapport au poids total de phénylalanine dans l'hydrolysat ;
au moins 95%, de préférence 100%, de lysine sous forme libre en poids par rapport au poids total de lysine dans l'hydrolysat ;
au moins 95%, de préférence 100%, d'arginine sous forme libre en poids par rapport au poids total d'arginine dans l'hydrolysat ;
au moins 95%, de préférence 100%, de proline sous forme libre en poids par rapport au poids total de proline dans l'hydrolysat.

Avantageusement au moins 94%, de préférence 96%, avantageusement 100% en poids des acides aminés présents dans l'hydrolysat présentent une masse molaire inférieure ou égale à 250 daltons, de préférence au moins 98% en poids des acides aminés présents dans l'hydrolysat présentent une masse molaire inférieure ou égale à 185 daltons.

De préférence, l'hydrolysat selon la présente invention comprend les acides aminés libres suivants, en poids, par rapport au poids total des acides aminés dudit hydrolysat :
de l'acide aspartique en une teneur allant de 1,00 à 7,50 %, de préférence allant de 1,00 à 3,00 %, et de manière préférée 1,89% en poids ;
de la thréonine en une teneur allant de 3,50 à 6,50 %, de préférence allant de 4,00 à 6,00 %, et de manière préférée 5,36% en poids ;
de la sérine en une teneur allant de 10,00 à 25,00 %, de préférence allant de 20,00 à 23,00 %, et de manière préférée 22,08 % en poids ;
de l'acide glutamique en une teneur allant de 2,00 à 10,50 %, de préférence allant de 2,00 à 4,00 %, et de manière préférée 2,84 % en poids ;
de la glycine en une teneur allant de 6,00 à 25,00 %, de préférence allant de 20,00 à 23,00 %, et de manière préférée 22,08 % en poids ;
de l'alanine en une teneur allant de 3,50 à 12,00 %, de préférence allant de 8,00 à 10,50 %, et de manière préférée 9,46% en poids ;
de la valine en une teneur allant de 4,00 à 8,50 %, de préférence allant de 4,00 à 5,00 %, et de manière préférée 4,42 % en poids ;
de la méthionine en une teneur allant de 0,10 à 2,00 %, de préférence allant de 0,20 à 1,00 %, et de manière préférée 0,32% en poids ;
de l'isoleucine en une teneur allant de 1,00 à 5,50 %, de préférence allant de 1,50 à 3,00 %, et de manière préférée 2,21% en poids ;
de la leucine en une teneur allant de 4,50 à 8,50 %, de préférence allant de 5,00 à 6,00 %, et de manière préférée 5,36 % en poids ;
de la tyrosine en une teneur allant de 0,2 à 2,0 %, de préférence allant de 0,3 à 1,00 %, et de manière préférée 0,32% en poids ;
de la phénylalanine en une teneur allant de 3,50 à 8,00 %, de préférence allant de 5,00 à 7,00 %, et de manière préférée 5,99% en poids ;
de la lysine en une teneur allant de 0,30 à 3,00 %, de préférence allant de 0,50 à 1,50 %, et de manière préférée 0,63% en poids ;
de l'histidine en une teneur allant de 0,2 à 5,0 %, de préférence allant de 0,50 à 1,50 %,et de manière préférée 0,63 % en poids ;
de l'arginine en une teneur allant de 2,50 à 6,50 %, de préférence allant de 3,00 à 4,00 %, et de manière préférée 3,79% en poids ;
de la proline en une teneur allant de 9,00 à 15,00 %, de préférence allant de 10,00 à 13,00 %, et de manière préférée 12,62% en poids.

Le procédé de préparation de l'hydrolysat de kératine selon l'invention met en œuvre au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton.

L'hydrolyse chimique de la kératine est réalisée au moyen d'un acide de préférence un acide fort choisi parmi les acides chlorhydrique, phosphorique et sulfurique, de manière préférée l'acide chlorhydrique.

De préférence, la concentration en acide va de 14 à 34% poids.

De préférence, le ratio pondéral acide/matière kératinique, en particulier le ratio pondéral acide/plumes, va de 3 à 5.

L'hydrolyse chimique est généralement effectuée pendant une durée allant de 1 heure à 24 heures, de préférence allant de 2 heures à 24 heures et de manière préférée allant de 6 à 20 heures à une température allant de 110 à 115°C.

Selon l'invention, l'hydrolyse chimique est effectuée en deux étapes :
- une première hydrolyse chimique réalisée à une température allant de 60 à 80°C pendant une période allant de 4 à 5 heures puis
- une deuxième hydrolyse chimique réalisée à une température allant de 100 à 115°C pendant une période allant de 5 à 8 heures, les deux hydrolyses pouvant être réalisées sans étape de pause intermédiaire ou en effectuant une étape de pause intermédiaire comprise entre 1 heure et 7 jours.

Plus précisément la première hydrolyse chimique est réalisée à 72°C pendant 4,5 heures et la deuxième hydrolyse chimique est réalisée à 107°C pendant 6 heures, une pause intermédiaire de 24 à 80 heures étant effectuées entre les deux hydrolyses chimiques.

Avantageusement, l'hydrolysat obtenu à l'issue de l'hydrolyse chimique, réalisée en une ou plusieurs étapes, comprend moins de 3, 5 % en mole de cystine par rapport au nombre total de moles d'acides aminés de l'hydrolysat.

L'hydrolyse chimique, réalisée en une ou plusieurs étapes, est suivie d'au moins les étapes suivantes dans cet ordre :
une étape d'extraction de la cystine et de la tyrosine,
une étape de dessalement, une étape de filtration.

Les étapes d'extraction de la cystine et de la tyrosine sont réalisées au moyen d'une base, de préférence choisie parmi l'hydroxyde de sodium, l'hydroxyde de potassium de préférence l'hydroxyde de sodium. Les étapes d'extraction de la cystine et de la tyrosine sont des étapes classiques dont la mise en œuvre relève des compétences de l'homme du métier.

Les étapes d'hydrolyse chimique et d'extraction de la cystine et de la tyrosine sont suivies d'étapes telles que décrites ci-dessous de purification de l'hydrolysat obtenu.

L'étape de dessalement vise à éliminer le chlorure de sodium ou le chlorure de potassium formé au cours de l'étape d'extraction de la tyrosine par ajout d'hydroxyde de sodium ou hydroxyde de potassium sur l'acide chlorhydrique. Cette étape de dessalement est de préférence effectuée par électrodialyse. L'électrodialyse est classiquement effectuée, en opposant à l'hydrolysat de l'eau pure, et en appliquant un courant électrique à l'empilement alterné de membranes anioniques et cationiques.

De préférence, l'hydrolysat selon la présente invention comprend moins de 2%, de préférence moins de 1 % en mole de tyrosine par rapport au nombre total de moles d'acides aminés de l'hydrolysat, de manière encore préférée, l'hydrolysat ne contient pas de tyrosine.

De préférence, l'hydrolysat selon la présente invention comprend moins de 2%, de préférence moins de 1% et de manière préférée moins de 0,5% en mole de cystine par rapport au nombre total de moles d'acides aminés de l'hydrolysat, de manière encore préférée, l'hydrolysat ne contient pas de cystine.

Avantageusement, l'hydrolysat selon l'invention ne contient ni tyrosine, ni cystine, les seules traces de ces acides aminés étant dues aux limites des conditions opératoires et du matériel mis en œuvre lors de l'étape d'extraction. En outre, dans la mesure où l'hydrolysat selon l'invention n'est pas obtenu suite à une réduction par électrolyse, il ne comprend pas de cystéine.

De manière surprenante et avantageuse les auteurs de la présente invention ont montré que la mise en œuvre d'une filtration dans les conditions particulières de l'invention permet d'atteindre un taux d'acides aminés libres élevé sans destruction ou dénaturation desdits acides aminés.

Selon l'invention la filtration est une nanofiltration qui est effectuée au moyen d'une membrane filtrante de calibre maximal 300 Da, de préférence de calibre maximal 250 Da et avantageusement une membrane de calibre allant de 100 à 250 Da, à une pression comprise entre 15 et 40 bar et à un débit allant de 300 à 900 L/h, de préférence allant de 400 à 800 L/h.

La nanofiltration consiste à faire circuler par une pompe à haute pression l'hydrolysat dessalé à travers une membrane judicieusement calibrée. La fraction passante, appelée perméat, contient les acides aminés libres d'intérêt, la fraction retenue, appelée rétentat, contient les peptides et les acides aminés résiduels. Le procédé est mis en oeuvre à partir d'une cuve avec une pompe à recirculation jusqu'à obtention d'une teneur stable en acides aminés dans le perméat.

Le procédé mis en œuvre selon l'invention permet l'obtention d'un hydrolysat comprenant au moins 94%, de préférence au moins 96%, avantageusement 100% en poids d'acides aminés libres par rapport au poids total des acides aminés de l'hydrolysat et dans lequel : au moins 93%, de préférence au moins 95%, de valine sous forme libre en poids par rapport au poids total de valine dansl'hydrolysat, au moins 90%, de préférence au moins 95%, d'isoleucine sous forme libre en poids par rapport au poids total d'isoleucine dans l'hydrolysat, au moins 95%, de préférence 100%, de leucine sous forme libre en poids par rapport au poids total de leucine dans l'hydrolysat .

En outre la majorité des acides aminés, notamment les acides aminés suivants la thréonine, la sérine, la glycine, l'alanine, la phénylalanine, la lysine, l'arginine et la proline, sont au moins à 95% sous forme libre par rapport au poids total dudit acide aminé dans l'hydrolysat.

Comme déjà mentionné, la présente invention vise l'utilisation, par voie orale, de la composition, notamment de l'hydrolysat de kératine, selon l'invention ou obtenu suivant le procédé de préparation selon l'invention en tant que matière première pour l'alimentation animale.

La présente invention vise encore une matière première comprenant de 80 à 100% d'un hydrolysat selon la présente invention et éventuellement des ingrédients additionnels comme des acides aminés libres additionnels, notamment au moins un acide aminé choisi parmi la cystine, l'arginine, l'acide glutamique et l'acide aspartique.

Avantageusement, à titre d'ingrédient additionnel, on peut ajouter de la L-cystine, ou encore d'autres acides aminés tel que l'arginine, l'acide glutamique et l'acide aspartique qui sont en partie retenus par la membrane lors de la nanofiltration.

On entend par « matière première », tous produits d'origine végétale ou animale, à l'état naturel, frais ou conservés, et dérivés de leur transformation industrielle, ainsi que substances organiques ou inorganiques, comprenant ou non des additifs, qui sont destinés à être utilisés pour l'alimentation des animaux par voie orale, soit directement tels quels, soit après transformation, pour la préparation d'aliments composés pour animaux ,ou en tant que supports des prémélanges (Directive 96/25/CE du Conseil du 29 avril 1996).

La matière première selon la présente invention est un mélange d'acides aminés destiné à être incorporé dans un aliment complet et équilibré ou à être utilisé en tant que complément alimentaire. Elle est en conséquence destinée à une administration par voie orale chez les animaux terrestres et/ou marins et/ou chez l'homme. Ladite matière première n'appartient pas au domaine thérapeutique.

La présente invention concerne plus particulièrement l'utilisation de l'hydrolysat, en alimentation animale et plus particulièrement en tant que matière première source d'acides aminés libres permettant de s'affranchir des protéines alimentaires d'origine végétale et/ou animale de structure moléculaire complexe et de poids moléculaire important.

De préférence, la matière première pour l'alimentation animale comprend en outre de la cystine.

De préférence, la matière première pour l'alimentation animale comprend en plus des acides amines de l'hydrolysat, au moins un acide aminé additionnel choisi parmi l'arginine, l'acide glutamique et l'acide aspartique.

De préférence, la matière première pour l'alimentation animale selon la présente invention comprend les acides aminés suivants, en poids, par rapport au poids total des acides aminés de la composition :
de l'acide aspartique en une teneur allant de 6,00 à 8,00 %, et de manière préférée 6,89 % en poids ;
de la thréonine en une teneur allant de 3,50 à 5,50 %, et de manière préférée 4,50% en poids ;
de la sérine en une teneur allant de 17,00 à 19,00 %, et de manière préférée 17,91 % en poids ;
de l'acide glutamique en une teneur allant de 9,00 à 11,00 %, et de manière préférée 9,84% en poids ;
de la glycine en une teneur allant de 17,00 à 19,00 %, et de manière préférée 17,90 % en poids ;
de l'alanine en une teneur allant de 7,00 à 9,00 %, et de manière préférée 7,95 % en poids ;
de la valine en une teneur allant de 3,00 à 5,00 %, et de manière préférée 3,71 % en poids ;
de la cystine en une teneur allant de 1,00 à 3,00 %, et de manière préférée 2,00 % en poids
de la méthionine en une teneur allant de 0,10 à 1,00 %, et de manière préférée 0,25 % en poids ;
de l'isoleucine en une teneur allant de 1,00 à 3,00 %, et de manière préférée 1,85 % en poids ;
de la leucine en une teneur allant de 3,50 à 5,50 %, et de manière préférée 4,53 % en poids ;
de la tyrosine en une teneur allant de 0,10 à 1,00 %, et de manière préférée 0,25% en poids ;
de la phénylalanine en une teneur allant de 4,00 à 6,00 %, et de manière préférée 5,03 % en poids ;
de la lysine en une teneur allant de 0,30 à 1,00 % et de manière préférée 0,50 % en poids ;
de l'histidine en une teneur allant de 0,30 à 1,00 % et de manière préférée 0,50 % en poids ;
de l'arginine en une teneur allant de 5,00 à 7,00 %, et de manière préférée 5,79 % en poids ;
de la proline en une teneur allant de 10,00 à 12,00 %, et de manière préférée 10,60 % en poids.

La formulation de la matière première pour l'alimentation animale conforme à l'invention met en œuvre des procédés classiques qui font partie des compétences générales de l'homme du métier.

Comme déja mentionné, la présente invention vise encore un aliment complet pour l'alimentation animale comprenant de 5 à 40 % en poids de la composition ou de préférence de l'hydrolysat selon l'invention par rapport au poids total dudit aliment complet.

Cet aliment complet est hautement anallergique.

L'aliment complet pour l'alimentation animale conforme à l'invention peut être formulé avec les excipients usuellement utilisés dans les compositions destinées à la voie orale, notamment les agents humectants, les épaississants, les agents de textures, les agents de saveur, les agents d'enrobage, les conservateurs, les antioxydants, les colorants, les extraits de plante, les ingrédients non protéiques tels que les amidons, les fibres végétales, les minéraux et les vitamines.

Bien entendu, l'homme du métier veillera à choisir ces excipients de manière à ne pas altérer les propriétés de l'aliment complet pour l'alimentation animale.

L'aliment complet pour l'alimentation animale conforme à l'invention peut être formulé selon une des présentations suivantes : une croquette, une gélule, une dragée, un comprimé, une capsule molle ou dure, ou encore une suspension, une solution, un gel, une préparation sèche contenant moins de 15% poids d'eau, ou humide comprenant au moins 50% poids d'eau et 85% poids d'eau au maximum.

La formulation de l'aliment complet pour l'alimentation animale conforme à l'invention met en œuvre des procédés classiques qui font partie des compétences générales de l'homme du métier.

L'invention vise aussi l'utilisation l'hydrolysat selon l'invention pour préparer une matière première ou un aliment complet pour l'alimentation animale.

Les exemples qui suivent visent à illustrer l'invention sans en limiter la portée.

### Exemples

### Exemple 1- Hydrolysat 1

### Préparation de l'hydrolysat 1

On introduit 4500 kg de plumes de volaille dans un réacteur/hydrolyseur de 20000 litres. Une première étape d'hydrolyse chimique est réalisée en ajoutant 18 000 litres d'acide chlorhydrique (24%), l'hydrolyse est effectuée à 72°C pendant 4,5 heures. Le produit obtenu est stocké pendant 48 heures à température ambiante (pause intermédiaire). Ensuite une deuxième hydrolyse chimique est effectuée en chauffant à 107°C pendant 6 heures sans ajout d'acide. Le produit obtenu est laissé à refroidir. La cystine et la tyrosine sont extraites par précipitation avec de l'hydroxyde de sodium à un pH compris entre 4 et 5. L'hydrolysat est dessalé par électrodialyse. On obtient 22000 kg d'hydrolysat 1 sous forme liquide.

### Exemple 2- Hydrolysat 2

### Préparation de l'hydrolysat 2

1 kg d'hydrolysat 1 dessalé est dilué par 19,2 kg d'eau purifiée et envoyé sous une pression de 20 bars avec un débit de 400 L/h à travers une membrane de nanofiltration calibrée à 100-250 Da. Le rétentat recueilli pèse 5,2 kg, le perméat 14,55 kg. L'hydrolysat dessalé entrant contient 93% d'acides aminés libres (soit 0,19 kg), le perméat sortant contient 99,06 % d'acides aminés libres, soit 0,05 kg. Le rendement en acides aminés libres est donc de 26,4 %.

### Exemple 3- Hydrolysat 3

### Préparation de l'hydrolysat 3

1 kg d'hydrolysat 1 dessalé est dilué par 12,6 kg d'eau purifiée et envoyé sous une pression de 36 bars avec un débit de 800 L/h à travers une membrane de nanofiltration calibrée à 100-250 Da. Le rétentat recueilli pèse 5,2 kg, le perméat 8,55 kg. L'hydrolysat dessalé entrant contient 93% d'acides aminés libres (soit 0,19 kg), le perméat sortant contient 97,87% d'acides aminés libres, soit 0,04 kg. Le rendement en acides aminés libres est donc de 22,4 %.

### Résultats

Le tableau 1 présente, pour chaque acide aminé présent, la fraction pondérale acides aminés libres / acides aminés totaux.

**[Tableau 1 ]**

| | **Hydrolysat 1 Non Conforme à l'invention** | **Hydrolysat 2 Conforme à l'invention** | **Hydrolysat 3 Conforme à l'invention** |
|---|---|---|---|
| Ac. Aspartique | 97,19% | 100,00% | 92,31% |
| Thréonine | 93,47% | 100,00% | 100,00% |
| Sérine | 100,00% | 100,00% | 100,00% |
| Ac. Glutamique | 92,33% | 90,00% | 100,00% |
| Glycine | 90,69% | 100,00% | 96,84% |
| Alanine | 98,66% | 100,00% | 94,87% |
| Valine | 69,57% | 96,55% | 94,74% |
| Cystine | 75,47% | * | * |
| Méthionine | 100,00% | * | 100,00% |
| Isoleucine | 76,59% | 93,33% | 90,00% |
| Leucine | 90,56% | 100,00% | 95,83% |
| Tyrosine | 71,75% | * | 100,00% |
| Phénylalanine | 95,15% | 100,00% | 100,00% |
| Lysine | 95,87% | 100,00% | 100,00% |
| Histidine | 100,00% | 100,00% | 80,00% |
| Arginine | 92,64% | 100,00% | 100,00% |
| Proline | 92,57% | 100,00% | 100,00% |
| **total** | **90,86%** | **99,06%** | **97.87%** |

| | | | |
|---|---|---|---|
| * pourcentage d'acide aminé sous forme libre inférieur à 0,002% dans l'hydrolysat. | | | |

### Détermination de la composition de l'hydrolysat 2

Les acides aminés de l'hydrolysat 2 sont dosés selon une méthode adaptée du règlement CE 152/2009.

Les acides aminés sont séparés par chromatographie (CLHP ou « HPLC » en langue anglaise) avec colonne échangeuse d'ions et dosés par réaction avec la ninhydrine et détection photométrique à 570 nm.

Dans le tableau 2, sont représentés pour chaque acide aminé de l'hydrolysat 2, la teneur en acides aminés libres par rapport au poids total des acides aminés.

**[Tableau 2 ]**

| Acide aminé | Teneur (% pds) dans la composition | Acide aminé | Teneur (% pds) dans la composition |
|---|---|---|---|
| Asp | 1.89 | Ile | 2.21 |
| Thr | 5.36 | Leu | 5.36 |
| Ser | 22.08 | Tyr | 0.32 |
| Glu | 2.84 | Phe | 5.99 |
| Gly | 22.08 | Lys | 0.63 |
| Ala | 9.46 | His | 0.63 |
| Val | 4.42 | Arg | 3.79 |
| L-Cystine | 0.00 | Pro | 12.62 |
| Met | 0.32 | **Total** | **100,00** |

### Exemple 4

### - Matière première pour l'alimentation animale

On prépare une matière première pour la nutrition animale à partir de la composition du tableau 2 à laquelle on ajoute de la L-cystine, de l'acide glutamique, de l'arginine et de l'acide aspartique de manière à obtenir la composition suivante présentée dans le tableau 3.

Dans le tableau 3, sont représentés pour chaque acide aminé, la teneur en acides aminés par rapport au poids total des acides aminés de la matière première pour alimentation animale.

**[Tableau 3 ]**

| Acide aminé | Teneur (% pds) dans la composition | Acide aminé | Teneur (% pds) dans la composition |
|---|---|---|---|
| Asp | 6.89 | Ile | 1.85 |
| Thr | 4.50 | Leu | 4.53 |
| Ser | 17.91 | Tyr | 0.25 |
| Glu | 9.84 | Phe | 5.03 |
| Gly | 17.90 | Lys | 0.50 |
| Ala | 7.95 | His | 0.50 |
| Val | 3.71 | Arg | 5.79 |
| L-Cystine | 2.00 | Pro | 10.60 |
| Met | 0.25 | **Total** | **100,00** |

Cette composition est hautement anallergique.

En outre son pouvoir d'appétence sur les chats et les chiens a été observé.

## Revendications

1. Hydrolysat de kératine **caractérisé en ce qu'**il comprend au moins 94 %, de préférence au moins 96 %, et de manière encore préférée 100% en poids d'acides aminés libres par rapport au poids total des acides aminés de l'hydrolysat ,
ledit hydrolysat comprenant au moins les acides aminés suivants :
au moins 93%, de préférence au moins 95%, de valine sous forme libre en poids par rapport au poids total de valine dans l'hydrolysat ,
au moins 90%, de préférence au moins 95%, d'isoleucine sous forme libre en poids par rapport au poids total d'isoleucine dans l'hydrolysat, et au moins 95%, de préférence 100%, de leucine sous forme libre en poids par rapport au poids total de leucine dans l'hydrolysat,
ledit hydrolysat étant susceptible d'être obtenu par un procédé de préparation dans lequel la matière kératinique est une matière kératinique de volaille,
comprenant au moins les étapes suivantes, dans cet ordre :
- soumettre la matière kératinique à au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton,
ladite hydrolyse chimique étant effectuée en deux étapes :
- une première hydrolyse chimique réalisée à une température allant de 60 à 80°C pendant une période allant de 4 à 5 heures puis
- une deuxième hydrolyse chimique réalisée à une température allant de 100 à 115°C pendant une période allant de 5 à 8 heures,
les deux hydrolyses pouvant être réalisées sans étape de pause intermédiaire ou en effectuant une étape de pause intermédiaire comprise entre 1 heure et 7 jours ;
- extraire la tyrosine et la cystine dudit hydrolysat, de préférence au moyen d'une base ;
- dessaler ledit hydrolysat ;
- effectuer une nanofiltration au moyen d'une membrane filtrante de calibre maximal 300 Da, à une pression comprise entre 15 et 40 bars et à un débit allant de 300 à 900 L/h
- éventuellement sécher.

2. Hydrolysat selon la revendication 1 **caractérisé en ce qu'**il comprend les acides aminés libres suivants :
au moins 95%, de préférence 100%, de thréonine sous forme libre en poids par rapport au poids total de thréonine dans l'hydrolysat;
au moins 95%, de préférence 100%, de sérine sous forme libre en poids par rapport au poids total de sérine dans l'hydrolysat;
au moins 95%, de préférence 100%, de glycine sous forme libre en poids par rapport au poids total de glycine dans l'hydrolysat;
au moins 95%, de préférence 100%, d'alanine sous forme libre en poids par rapport au poids total d'alanine dans l'hydrolysat;
au moins 95%, de préférence 100%, de phénylalanine sous forme libre en poids par rapport au poids total de phénylalanine dans l'hydrolysat ;
au moins 95%, de préférence 100%, de lysine sous forme libre en poids par rapport au poids total de lysine dans l'hydrolysat;
au moins 95%, de préférence 100%, d'arginine sous forme libre en poids par rapport au poids total d'arginine dans l'hydrolysat;
au moins 95%, de préférence 100%, de proline sous forme libre en poids par
rapport au poids total de proline dans l'hydrolysat.

3. Hydrolysat selon la revendication 1 ou 2 **caractérisé en ce qu'**il comprend moins de 2%, de préférence moins de 1 % et de manière encore préférée moins de 0,5 % en mole de cystine par rapport au nombre total de moles d'acides aminés de l'hydrolysat.

4. Hydrolysat selon l'une quelconque des revendications précédentes **caractérisé en ce qu'**il comprend les acides aminés suivants, en poids, par rapport au poids total des acides aminés de l'hydrolysat :
de l'acide aspartique en une teneur allant de 1,00 à 7,50 %, de préférence allant de 1,00 à 3,00 %, et de manière préférée 1,89% en poids ;
de la thréonine en une teneur allant de 3,50 à 6,50 %, de préférence allant de 4,00 à 6,00 %, et de manière préférée 5,36% en poids ;
de la sérine en une teneur allant de 10,00 à 25,00 %, de préférence allant de 20,00 à 23,00 %, et de manière préférée 22,08 % en poids ;
de l'acide glutamique en une teneur allant de 2,00 à 10,50 %, de préférence allant de 2,00 à 4,00 %, et de manière préférée 2,84 % en poids ;
de la glycine en une teneur allant de 6,00 à 25,00 %, de préférence allant de 20,00 à 23,00 %, et de manière préférée 22,08 % en poids ;
de l'alanine en une teneur allant de 3,50 à 12,00 %, de préférence allant de 8,00 à 10,50 %, et de manière préférée 9,46% en poids ;
de la valine en une teneur allant de 4,00 à 8,50 %, de préférence allant de 4,00 à 5,00 %, et de manière préférée 4,42 % en poids ;
de la méthionine en une teneur allant de 0,10 à 2,00 %, de préférence allant de 0,20 à 1,00 %, et de manière préférée 0,32% en poids ;
de l'isoleucine en une teneur allant de 1,00 à 5,50 %, de préférence allant de 1,50 à 3,00 %, et de manière préférée 2,21% en poids ;
de la leucine en une teneur allant de 4,50 à 8,50 %, de préférence allant de 5,00 à 6,00 %, et de manière préférée 5,36 % en poids ;
de la tyrosine en une teneur allant de 0,2 à 2,0 %, de préférence allant de 0,3 à 1,00 %, et de manière préférée 0,32% en poids ;
de la phénylalanine en une teneur allant de 3,50 à 8,00 %, de préférence allant de 5,00 à 7,00 %, et de manière préférée 5,99% en poids ;
de la lysine en une teneur allant de 0,30 à 3,00 %, de préférence allant de 0,50 à 1,50 %, et de manière préférée 0,63% en poids ;
de l'histidine en une teneur allant de 0,2 à 5,0 %, de préférence allant de 0,50 à 1,50 %,et de manière préférée 0,63 % en poids ;
de l'arginine en une teneur allant de 2,50 à 6,50 %, de préférence allant de 3,00 à 4,00 %, et de manière préférée 3,79% en poids ;
de la proline en une teneur allant de 9,00 à 15,00 %, de préférence allant de 10,00 à 13,00 %, et de manière préférée 12,62% en poids

5. Procédé de préparation de l'hydrolysat selon l'une
quelconque des revendications précédentes dans lequel la matière kératinique est une matière kératinique de volaille, **caractérisé en ce qu'**il comprend au moins les étapes suivantes, dans cet ordre :
- soumettre la matière kératinique à au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton,
ladite hydrolyse chimique étant effectuée en deux étapes :
- une première hydrolyse chimique réalisée à une température allant de 60 à 80°C pendant une période allant de 4 à 5 heures puis
- une deuxième hydrolyse chimique réalisée à une température allant de 100 à 115°C pendant une période allant de 5 à 8 heures,
les deux hydrolyses pouvant être réalisées sans étape de pause intermédiaire ou en effectuant une étape de pause intermédiaire comprise entre 1 heure et 7 jours ;
- extraire la tyrosine et la cystine dudit hydrolysat, de préférence au moyen d'une base ;
- dessaler ledit hydrolysat ;
- effectuer une nanofiltration au moyen d'une membrane filtrante de calibre maximal 300 Da, à une pression comprise entre 15 et 40 bars et à un débit allant de 300 à 900 L/h
- éventuellement sécher.

6. Procédé de préparation de l'hydrolysat selon la revendication 5 dans lequel l'hydrolyse chimique est effectuée au moyen d'un acide fort choisi parmi les acides chlorhydrique, phosphorique et sulfurique, de manière préférée l'acide chlorhydrique.

7. Procédé de préparation de l'hydrolysat l'une quelconque des revendications 5 ou 6 dans lequel la concentration en acide va de 14 à 34% poids.

8. Procédé de préparation de l'hydrolysat l'une quelconque des revendications 5 à 7 dans lequel le ratio pondéral acide/matière kératinique va de 3 à 5.

9. Matière première pour l'alimentation animale comprenant de 80 à 100 % en poids de l'hydrolysat selon l'une quelconque des revendications 1 à 4 ou préparé suivant le procédé selon l'une quelconque des revendications 5 à 8, par rapport au poids total de ladite matière première.

10. Matière première pour l'alimentation animale selon la revendication 9 **caractérisée en ce qu'**elle comprend en outre de la cystine.

11. Matière première pour l'alimentation animale selon l'une quelconque des revendications 9 ou 10 **caractérisée en ce qu'**elle comprend en plus des acides aminés de l'hydrolysat selon l'une quelconque des revendications 1 à 4, au moins un acide aminé additionnel choisi parmi l'arginine, l'acide glutamique et l'acide aspartique.

12. Matière première pour l'alimentation animale selon l'une quelconque des revendications 9 à 11
**caractérisée en ce qu'**elle comprend les acides aminés suivants, en poids, par rapport au poids total des acides aminés de la composition :
de l'acide aspartique en une teneur allant de 6,00 à 8,00 %, et de manière préférée 6,89 % en poids ;
de la thréonine en une teneur allant de 3,50 à 5,50 %, et de manière préférée 4,50% en poids ;
de la sérine en une teneur allant de 17,00 à 19,00 %, et de manière préférée 17,91 % en poids ;
de l'acide glutamique en une teneur allant de 9,00 à 11,00 %, et de manière préférée 9,84% en poids ;
de la glycine en une teneur allant de 17,00 à 19,00 %, et de manière préférée 17,90 % en poids ;
de l'alanine en une teneur allant de 7,00 à 9,00 %, et de manière préférée 7,95 % en poids ;
de la valine en une teneur allant de 3,00 à 5,00 %, et de manière préférée 3,71 % en poids ;
de la cystine en une teneur allant de 1,00 à 3,00 %, et de manière préférée 2,00 % en poids
de la méthionine en une teneur allant de 0,10 à 1,00 %, et de manière préférée 0,25 % en poids ;
de l'isoleucine en une teneur allant de 1,00 à 3,00 %, et de manière préférée 1,85 % en poids ;
de la leucine en une teneur allant de 3,50 à 5,50 %, et de manière préférée 4,53 % en poids ;
de la tyrosine en une teneur allant de 0,10 à 1,00 %, et de manière préférée 0,25% en poids ;
de la phénylalanine en une teneur allant de 4,00 à 6,00 %, et de manière préférée 5,03 % en poids ;
de la lysine en une teneur allant de 0,30 à 1,00 % et de manière préférée 0,50 % en poids ;
de l'histidine en une teneur allant de 0,30 à 1,00 % et de manière préférée 0,50 % en poids ;
de l'arginine en une teneur allant de 5,00 à 7,00 %, et de manière préférée 5,79 % en poids ;
de la proline en une teneur allant de 10,00 à 12,00 %, et de manière préférée 10,60 % en poids.

13. Aliment complet pour l'alimentation animale comprenant de 5 à 40 % en poids de l'hydrolysat selon l'une quelconque des revendications 1 à 4 ou préparé suivant le procédé selon l'une quelconque des revendications 5 à 8.

## Patentansprüche

1. Keratinhydrolysat, **dadurch gekennzeichnet, dass** es
einen Gewichtsanteil freier Aminosäuren von mindestens 94 %, vorzugsweise mindestens 96 % und noch mehr bevorzugt 100 % bezogen auf das Gesamtgewicht der Aminosäuren im Hydrolysat aufweist,
wobei das Hydrolysat mindestens die folgenden Aminosäuren aufweist:
einen Gewichtsanteil von freiem Valin von mindestens 93 %, vorzugsweise mindestens 95 % bezogen auf das Gesamtgewicht des Valins im Hydrolysat,
einen Gewichtsanteil von freiem Isoleucin von mindestens 90 %,
vorzugsweise mindestens 95 % bezogen auf das Gesamtgewicht des Isoleucins im Hydrolysat und einen Gewichtsanteil von freiem Leucin von mindestens 95 %, vorzugsweise 100 % bezogen auf das Gesamtgewicht des Leucins im Hydrolysat,
wobei das Hydrolysat durch ein Zubereitungsverfahren hergestellt werden kann, bei dem aus Geflügel gewonnenes Keratinmaterial verwendet wird und
das mindestens die folgenden Schritte in dieser Reihenfolge umfasst:
- das Keratinmaterial mittels einer Säure mindestens einer chemischen Hydrolyse unter Bedingungen aussetzen, die geeignet sind, ein Hydrolysat zu erhalten, welches einen Gewichtsanteil von freien Aminosäuren von mindestens 88 % bezogen auf das Gesamtgewicht der Aminosäuren des Hydrolysats aufweist, wobei die übrigen Aminosäuren des Hydrolysats in Form von Peptiden mit einer Molekularmasse kleiner oder gleich 800 Dalton vorliegen,
wobei die chemische Hydrolyse in zwei Schritten durchgeführt wird:
- eine erste chemische Hydrolyse, die bei einer Temperatur von 60 bis 80 °C über einen Zeitraum von 4 bis 5 Stunden durchgeführt wird, dann
- eine zweite chemische Hydrolyse, die bei einer Temperatur von 100 bis 115 °C über einen Zeitraum von 5 bis 8 Stunden durchgeführt wird,
wobei die beiden Hydrolysen ohne Zwischenpausenschritt oder mit einem Zwischenpausenschritt zwischen 1 Stunde und 7 Tagen durchgeführt werden können;
- Tyrosin und Zystin aus dem Hydrolysat extrahieren, vorzugsweise mittels einer Base;
- das Hydrolysat entsalzen;
- eine Nanofiltration mittels einer Filtermembran mit einer maximalen Größe von 300 Da bei einem Druck zwischen 15 und 40 bar und einem Durchfluss von 300 bis 900 l/h durchführen
- ggf. trocknen.

2. Hydrolysat nach Anspruch 1, **dadurch gekennzeichnet, dass** es folgende freie Aminosäuren aufweist:
einen Gewichtsanteil von freiem Threonin von mindestens 95 %,
vorzugsweise 100 % bezogen auf das Gesamtgewicht des Threonins im Hydrolysat;
einen Gewichtsanteil von freiem Serin von mindestens 95 %, vorzugsweise 100 % bezogen auf das Gesamtgewicht des Serins im Hydrolysat;
einen Gewichtsanteil von freiem Glycin von mindestens 95 %, vorzugsweise 100 % bezogen auf das Gesamtgewicht des Glycins im Hydrolysat;
einen Gewichtsanteil von freiem Alanin von mindestens 95 %, vorzugsweise 100 % bezogen auf das Gesamtgewicht des Alanins im Hydrolysat;
einen Gewichtsanteil von freiem Phenylalanin von mindestens 95 %,
vorzugsweise 100 % bezogen auf das Gesamtgewicht des Phenylalanins im Hydrolysat;
einen Gewichtsanteil von freiem Lysin von mindestens 95 %, vorzugsweise 100 % bezogen auf das Gesamtgewicht des Lysins im Hydrolysat;
einen Gewichtsanteil von freiem Arginin von mindestens 95 %, vorzugsweise 100 % bezogen auf das Gesamtgewicht des Arginins im Hydrolysat;
einen Gewichtsanteil von freiem Prolin von mindestens 95 %, vorzugsweise 100 % bezogen auf das Gesamtgewicht des Prolins im Hydrolysat.

3. Hydrolysat nach Anspruch 1 oder 2, **dadurch gekennzeichnet, dass** der Stoffmengenanteil von Cystin weniger als 2 Mol-%, vorzugsweise weniger als 1 Mol-% und noch mehr bevorzugt weniger als 0,5 Mol-% bezogen auf die Gesamtstoffmenge der Aminosäuren des Hydrolysats umfasst.

4. Hydrolysat nach einem der vorhergehenden Ansprüche, **dadurch gekennzeichnet, dass** es die folgenden Gewichtsanteile von Aminosäuren bezogen auf das Gesamtgewicht der Aminosäuren des Hydrolysats aufweist:
Aspartinsäure mit einem Gewichtsanteil von 1,00 bis 7,50 %, vorzugsweise 1,00 bis 3,00 % und bevorzugt 1,89 %;
Threonin mit einem Gewichtsanteil von 3,50 bis 6,50 %, vorzugsweise 4,00 bis 6,00 % und bevorzugt 5,36 %;
Serin mit einem Gewichtsanteil von 10,00 bis 25,00 %, vorzugsweise 20,00 bis 23,00 % und bevorzugt 22,08 %;
Glutaminsäure mit einem Gewichtsanteil von 2,00 bis 10,50 %, vorzugsweise 2,00 bis 4,00 % und bevorzugt 2,84 %;
Glycin mit einem Gewichtsanteil von 6,00 bis 25,00 %, vorzugsweise 20,00 bis 23,00 % und bevorzugt 22,08 %;
Alanin mit einem Gewichtsanteil von 3,50 bis 12,00 % vorzugsweise 8,00 bis 10,50 % und bevorzugt 9,46 %;
Valin mit einem Gewichtsanteil von 4,00 bis 8,50 %, vorzugsweise 4,00 bis 5,00 % und bevorzugt 4,42 %;
Methionin mit einem Gewichtsanteil von 0,10 bis 2,00 %, vorzugsweise 0,20 bis 1,00 % und bevorzugt 0,32 %;
Isoleucin mit einem Gewichtsanteil von 1,00 bis 5,50 % vorzugsweise 1,50 bis 3,00 % und bevorzugt 2,21 %;
Leucin mit einem Gewichtsanteil von 4,50 bis 8,50 %, vorzugsweise 5,00 bis 6,00 % und bevorzugt 5,36 %;
Tyrosin mit einem Gewichtsanteil von 0,2 bis 2,0 %, vorzugsweise 0,3 bis 1,00 % und bevorzugt 0,32 %;
Phenylalanin mit einem Gewichtsanteil von 3,50 bis 8,00 %, vorzugsweise 5,00 bis 7,00 % und bevorzugt 5,99 %;
Lysin mit einem Gewichtsanteil von 0,30 bis 3,00 %, vorzugsweise 0,50 bis 1,50 % und bevorzugt 0,63 %;
Histidin mit einem Gewichtsanteil von 0,2 bis 5,0 % vorzugsweise 0,50 bis 1,50 % und bevorzugt 0,63 %;
Arginin mit einem Gewichtsanteil von 2,50 bis 6,50 % vorzugsweise 3,00 bis 4,00 % und bevorzugt 3,79 %;
Prolin mit einem Gewichtsanteil von 9,00 bis 15,00 %, vorzugsweise 10,00 bis 13,00 % und bevorzugt 12,62 %.

5. Verfahren zur Zubereitung des Hydrolysats nach einem
der vorhergehenden Ansprüche, wobei das Keratinmaterial ein aus Geflügel gewonnenes Keratinmaterial ist, **dadurch gekennzeichnet, dass** es mindestens die folgenden Schritte in dieser Reihenfolge umfasst:
- das Keratinmaterial mittels einer Säure mindestens einer chemischen Hydrolyse unter Bedingungen aussetzen, die geeignet sind, ein Hydrolysat zu erhalten, welches einen Gewichtsanteil von freien Aminosäuren von mindestens 88 % bezogen auf das Gesamtgewicht der Aminosäuren des Hydrolysats aufweist, wobei die übrigen Aminosäuren des Hydrolysats in Form von Peptiden mit einer Molekularmasse kleiner oder gleich 800 Dalton vorliegen,
wobei die chemische Hydrolyse in zwei Schritten durchgeführt wird:
- eine erste chemische Hydrolyse, die bei einer Temperatur von 60 bis 80 °C über einen Zeitraum von 4 bis 5 Stunden durchgeführt wird, dann
- eine zweite chemische Hydrolyse, die bei einer Temperatur von 100 bis 115 °C über einen Zeitraum von 5 bis 8 Stunden durchgeführt wird,
wobei die beiden Hydrolysen ohne Zwischenpausenschritt oder mit einem Zwischenpausenschritt zwischen 1 Stunde und 7 Tagen durchgeführt werden können;
- Tyrosin und Zystin aus dem Hydrolysat extrahieren, vorzugsweise mittels einer Base;
- das Hydrolysat entsalzen;
- eine Nanofiltration mittels einer Filtermembran mit einer maximalen Größe von 300 Da bei einem Druck zwischen 15 und 40 bar und einem Durchfluss von 300 bis 900 l/h durchführen
- ggf. trocknen.

6. Verfahren zur Zubereitung des Hydrolysats nach Anspruch 5, wobei die chemische Hydrolyse mittels einer starken Säure durchgeführt wird, die aus Salz-, Phosphor- und Schwefelsäure, vorzugsweise Salzsäure, ausgewählt wird.

7. Verfahren zur Zubereitung des Hydrolysats nach einem der Ansprüche 5 oder 6, wobei die Säurekonzentration bei einem Gewichtsanteil von 14 bis 34 % liegt.

8. Verfahren zur Zubereitung des Hydrolysats nach einem der Ansprüche 5 bis 7, wobei das Gewichtsverhältnis Säure/Keratinmaterial bei 3 bis 5 liegt.

9. Futtermittel-Rohstoff, der einen Gewichtsanteil von 80 bis 100 % Hydrolysat nach einem der Ansprüche 1 bis 4 oder zubereitet gemäß dem Verfahren einer der Ansprüche 5 bis 8 bezogen auf das Gesamtgewicht des Rohstoffs aufweist.

10. Futtermittel-Rohstoff nach Anspruch 9, **dadurch gekennzeichnet, dass** er ferner Zystin enthält.

11. Futtermittel-Rohstoff nach einem der Ansprüche 9 oder 10, **dadurch gekennzeichnet, dass** er zusätzlich zu den Aminosäuren des Hydrolysats nach einem der Ansprüche 1 bis 4 mindestens eine zusätzliche Aminosäure umfasst, die aus Arginin, Glutaminsäure und Aspartinsäure ausgewählt wird.

12. Futtermittel-Rohstoff nach einem der Ansprüche 9 bis 11, **dadurch gekennzeichnet, dass** er die folgenden Gewichtsanteile von Aminosäuren bezogen auf das Gesamtgewicht der Aminosäuren der Zusammensetzung aufweist:
Aspartinsäure mit einem Gewichtsanteil von 6,00 bis 8,00 %, vorzugsweise 6,89 %;
Threonin mit einem Gewichtsanteil von 3,50 bis 5,50 %, vorzugsweise 4,50 %;
Serin mit einem Gewichtsanteil von 17,00 bis 19,00 %, vorzugsweise 17,91 %;
Glutaminsäure mit einem Gewichtsanteil von 9,00 bis 11,00 %, vorzugsweise 9,84 %;
Glycin mit einem Gewichtsanteil von 17,00 bis 19,00 %, vorzugsweise 17,90 %;
Alanin mit einem Gewichtsanteil von 7,00 bis 9,00 %, vorzugsweise 7,95 %;
Valin mit einem Gewichtsanteil von 3,00 bis 5,00 %, vorzugsweise 3,71 %;
Zystin mit einem Gewichtsanteil von 1,00 bis 3,00 %, vorzugsweise 2,00 %;
Methionin mit einem Gewichtsanteil von 0,10 bis 1,00 %, vorzugsweise 0,25 %;
Isoleucin mit einem Gewichtsanteil von 1,00 bis 3,00 %, vorzugsweise 1,85 %;
Leucin mit einem Gewichtsanteil von 3,50 bis 5,50 %, vorzugsweise 4,53 %;
Tyrosin mit einem Gewichtsanteil von 0,10 bis 1,00 %, vorzugsweise 0,25 %;
Phenylalanin mit einem Gewichtsanteil von 4,00 bis 6,00 %, vorzugsweise 5,03 %);
Lysin mit einem Gewichtsanteil von 0,30 bis 1,00 %, vorzugsweise 0,50 %;
Histidin mit einem Gewichtsanteil von 0,30 bis 1,00 %, vorzugsweise 0,50 %;
Arginin mit einem Gewichtsanteil von 5,00 bis 7,00 %, vorzugsweise 5,79 %;
Prolin mit einem Gewichtsanteil von 10,00 bis 12,00 %, vorzugsweise 10,60 %;

13. Vollfuttermittel mit einem Gewichtsanteil von 5 bis 40 % Hydrolysat nach einem der Ansprüche 1 bis 4 oder zubereitet gemäß dem Verfahren einer der Ansprüche 5 bis 8.

## Claims

1. Keratin hydrolysate **characterized in that** it comprises at least 94%, preferably at least 96%, and more preferably 100% by weight of free amino acids relative to the total weight of the amino acids of the hydrolysate, said hydrolysate comprising at least the following amino acids:
at least 93%, preferably at least 95%, of valine in free form by weight relative to the total weight of valine in the hydrolysate,
at least 90%, preferably at least 95%, of isoleucine in free form by weight relative to the total weight of isoleucine in the hydrolysate, and
at least 95%, preferably 100%, of leucine in free form by weight relative to the total weight of leucine in the hydrolysate,
said hydrolysate being obtainable by a preparation method wherein the keratin material is a poultry keratin material, comprising at least the following steps, in this order:
- subjecting the keratin material to at least one chemical hydrolysis by means of an acid under conditions suitable for obtaining a hydrolysate comprising at least 88% by weight of free amino acids relative to the total weight of the amino acids of the hydrolysate, the remainder of the amino acids of the hydrolysate being in the form of peptides having a molecular weight of less than or equal to 800 Daltons, the chemical hydrolysis being carried out in two steps:
- a first chemical hydrolysis carried out at a temperature ranging from 60 to 80°C for a period ranging from 4 to 5 hours, then
- a second chemical hydrolysis carried out at a temperature ranging from 100 to 115°C for a period ranging from 5 to 8 hours,
it being possible for the two hydrolyses to be carried out without an intermediate pause step or by performing an intermediate pause step of between 1 hour and 7 days
- extracting the tyrosine and the cystine from said hydrolysate, preferably by means of a base;
- desalifying said hydrolysate;
- carrying out a nanofiltration by means of a filtering membrane with a maximum calibre of 300 Da, at a pressure of between 15 and 40 bar and at a flow rate ranging from 300 to 900 I/h;
- optionally drying.

2. Hydrolysate according to Claim 1, **characterized in that** it comprises the following free amino acids:
at least 95%, preferably 100%, of threonine in free form by weight relative to the total weight of threonine in the hydrolysate;
at least 95%, preferably 100%, of serine in free form by weight relative to the total weight of serine in the hydrolysate;
at least 95%, preferably 100%, of glycine in free form by weight relative to the total weight of glycine in the hydrolysate;
at least 95%, preferably 100%, of alanine in free form by weight relative to the total weight of alanine in the hydrolysate;
at least 95%, preferably 100%, of phenylalanine in free form by weight relative to the total weight of phenylalanine in the hydrolysate;
at least 95%, preferably 100%, of lysine in free form by weight relative to the total weight of lysine in the hydrolysate;
at least 95%, preferably 100%, of arginine in free form by weight relative to the total weight of arginine in the hydrolysate;
at least 95%, preferably 100%, of proline in free form by weight relative to the total weight of proline in the hydrolysate.

3. Hydrolysate according to Claim 1 or 2, **characterized in that** it comprises less than 2 mol%, preferably less than 1 mol% and more preferably less than 0.5 mol% of cystine relative to the total number of moles of amino acids of the hydrolysate.

4. Hydrolysate according to any one of the preceding claims, **characterized in that** it comprises the following amino acids, by weight, relative to the total weight of the amino acids of the hydrolysate:
aspartic acid in a content ranging from 1.00 to 7.50%, preferably ranging from 1.00 to 3.00%, and of preferably 1.89% by weight;
threonine in a content ranging from 3.50 to 6.50%, preferably ranging from 4.00 to 6.00%, and of preferably 5.36% by weight;
serine in a content ranging from 10.00 to 25.00%, preferably ranging from 20.00 to 23.00%, and of preferably 22.08% by weight;
glutamic acid in a content ranging from 2.00 to 10.50%, preferably ranging from 2.00 to 4.00%, and of preferably 2.84% by weight;
glycine in a content ranging from 6.00 to 25.00%, preferably ranging from 20.00 to 23.00%, and of preferably 22.08% by weight;
alanine in a content ranging from 3.50 to 12.00%, preferably ranging from 8.00 to 10.50%, and of preferably 9.46% by weight;
valine in a content ranging from 4.00 to 8.50%, preferably ranging from 4.00 to 5.00%,
and of preferably 4.42% by weight;
methionine in a content ranging from 0.10 to 2.00%, preferably ranging from 0.20 to 1.00%, and of preferably 0.32% by weight;
isoleucine in a content ranging from 1.00 to 5.50%, preferably ranging from 1.50 to 3.00%, and of preferably 2.21% by weight;
leucine in a content ranging from 4.50 to 8.50%, preferably ranging from 5.00 to 6.00%, and of preferably 5.36% by weight;
tyrosine in a content ranging from 0.2 to 2.0%, preferably ranging from 0.3 to 1.00%, and of preferably 0.32% by weight;
phenylalanine in a content ranging from 3.50 to 8.00%, preferably ranging from 5.00 to 7.00%, and of preferably 5.99% by weight;
lysine in a content ranging from 0.30 to 3.00%, preferably ranging from 0.50 to 1.50%,
and of preferably 0.63% by weight;
histidine in a content ranging from 0.2 to 5.0%, preferably ranging from 0.50 to 1.50%,
and of preferably 0.63% by weight;
arginine in a content ranging from 2.50 to 6.50%, preferably ranging from 3.00 to 4.00%, and of preferably 3.79% by weight;
proline in a content ranging from 9.00 to 15.00%, preferably ranging from 10.00 to 13.00%, and of preferably 12.62% by weight.

5. Method for preparing the hydrolysate according to any one of the preceding claims, in which the keratin material is a poultry keratin material, **characterized in that** it comprises at least the following steps, in this order:
- subjecting the keratin material to at least one chemical hydrolysis by means of an acid under conditions suitable for obtaining a hydrolysate comprising at least 88% by weight of free amino acids relative to the total weight of the amino acids of the hydrolysate, the remainder of the amino acids of the hydrolysate being in the form of peptides having a molecular weight of less than or equal to 800 Daltons,
said chemical hydrolysis being carried out in two steps:
- a first chemical hydrolysis carried out at a temperature ranging from 60 to 80°C for a period ranging from 4 to 5 hours, then
- a second chemical hydrolysis carried out at a temperature ranging from 100 to 115°C for a period ranging from 5 to 8 hours,
it being possible for the two hydrolyses to be carried out without an intermediate pause step or by performing an intermediate pause step of between 1 hour and 7 days
- extracting the tyrosine and the cystine from said hydrolysate, preferably by means of a base;
- desalifying said hydrolysate;
- carrying out a nanofiltration by means of a filtering membrane with a maximum calibre of 300 Da, at a pressure of between 15 and 40 bar and at a flow rate ranging from 300 to 900 I/h;
- optionally drying.

6. Method for preparing the hydrolysate according to Claim 5, in which the chemical hydrolysis is carried out by means of a strong acid chosen from hydrochloric acid, phosphoric acid and sulfuric acid, preferably hydrochloric acid.

7. Method for preparing the hydrolysate according to Claim 5 or 6, in which the acid concentration ranges from 14 to 34% weight.

8. Method for preparing the hydrolysate according to any one of Claims 5 to 7, in which the acid to feather weight ratio preferably ranges from 3 to 5.

9. Starting material for animal feed, comprising from 80 to 100% by weight of the hydrolysate according to any one of Claims 1 to 4 or prepared according to the method according to any one of Claims 5 to 8, relative to the total weight of said starting material.

10. Starting material for animal feed according to Claim 9, **characterized in that** it also comprises cystine.

11. Starting material for animal feed according to any one of Claims 9 and 10, **characterized in that** it comprises, in addition to the amino acids of the hydrolysate according to any one of Claims 1 to 4, at least one additional amino acid chosen from arginine, glutamic acid and aspartic acid.

12. Starting material for animal feed according to any one of Claims 9 to 11, **characterized in that** it comprises the following amino acids, by weight, relative to the total weight of the amino acids of the composition:
aspartic acid in a content ranging from 6.00 to 8.00%, and of preferably 6.89% by weight;
threonine in a content ranging from 3.50 to 5.50%, and of preferably 4.50% by weight;
serine in a content ranging from 17.00 to 19.00%, and of preferably 17.91% by weight;
glutamic acid in a content ranging from 9.00 to 11.00%, and of preferably 9.84% by weight;
glycine in a content ranging from 17.00 to 19.00%, and of preferably 17.90% by weight;
alanine in a content ranging from 7.00 to 9.00%, and of preferably 7.95% by weight;
valine in a content ranging from 3.00 to 5.00%, and of preferably 3.71% by weight;
cystine in a content ranging from 1.00 to 3.00%, and of preferably 2.00% by weight;
methionine in a content ranging from 0.10 to 1.00%, and of preferably 0.25% by weight;
isoleucine in a content ranging from 1.00 to 3.00%, and of preferably 1.85% by weight;
leucine in a content ranging from 3.50 to 5.50%, and of preferably 4.53% by weight;
tyrosine in a content ranging from 0.10 to 1.00%, and of preferably 0.25% by weight;
phenylalanine in a content ranging from 4.00 to 6.00%, and of preferably 5.03% by weight;
lysine in a content ranging from 0.30 to 1.00%, and of preferably 0.50% by weight;
histidine in a content ranging from 0.30 to 1.00%, and of preferably 0.50% by weight;
arginine in a content ranging from 5.00 to 7.00%, and of preferably 5.79% by weight;
proline in a content ranging from 10.00 to 12.00%, and of preferably 10.60% by weight.

13. Complete feed for animal feed, comprising from 5 to 40% by weight of the hydrolysate according to any one of Claims 1 to 4 or prepared according to any one of Claims 5 to 8.
